# EUROPEAN PATENT SPECIFICATION

(11) **EP 1 714 976 B1**
(45) Date of publication and mention of the grant of the patent: **13.07.2011**
(21) Application number: 04820941.5
(22) Date of filing: 26.11.2004
(51) Int. Cl.: C07K 7/08, C07K 14/47, A61P 29/00, A61P 37/06

(54) **AMINO ACID SEQUENCES FROM ACTIVE PRINCIPLE OF MUSK AND THEIR ACETIC SALT, ALSO THE PREPARATION AND USAGE**
AMINOSÄURESEQUENZEN DES AKTIVEN PRINZIPS VON MOSCHUS UND DEREN ESSIGSÄURESALZ SOWIE DIE HERSTELLUNG UND VERWENDUNG
SEQUENCES D'ACIDES AMINES PROVENANT D'UN PRINCIPE ACTIF DU MUSC ET SEL ACETIQUE DE CES SEQUENCES, PROCEDE DE PREPARATION ET D'UTILISATION DE CES SEQUENCES D'ACIDES AMINES

(30) Priority: 05.01.2004 CN 200410000851
(43) Date of publication of application: 25.10.2006
(73) Proprietor: Wu, Wenyan, Guangzhou, Guangdong 510275 (CN)
(72) Inventor: Wu, Wenyan, Guangzhou, Guangdong 510275 (CN)
(74) Representative: Craven, Ian
(86) International application number: PCT/CN2004/001362
(87) International publication number: WO 2005/066201

(56) References cited:
- WO-A-02/26781
- LIU X.M. ET AL.: 'Studies on the antinflammatory protein of musk.i.isolation purification and properties.' ACTA ZOOLOGICA SINICA. vol. 38, no. 3, September 1992, pages 302 - 308, XP008071205
- ZHU X. ET AL.: 'The pharmacological activities of musk II. the anti-' ACTA PHARMACEUTICA SINICA. vol. 23, no. 6, 1988, pages 406 - 410, XP008071129
- XIAO Q.Z. ET AL.: 'Adult rat and human bone marrow mesenchymal stem cells differe' CHINESE JOURNAL OF PATHOPHYSIOLOGY. vol. 18, no. 10, 2002, pages 1179 - 1182, XP008071247
- WANG W. ET AL.: 'Effect of musk on certain functions of rat' CHINESE TRADITIONAL MEDICINE. vol. 29, no. 5, 1998, pages 322 - 324, XP008071135
- WANG W. ET AL.: 'Effects of the glucoprotein component of musk' ACTA ACADEMIAE MEDICINAE SINICAE. vol. 19, no. 3, June 1997, pages 222 - 226, XP008071128
- WANG W. ET AL.: 'Effects of the glucoprotein component of musk on func' CHINA JOURNAL OF CHINESE MATERIAL MEDICA. vol. 23, no. 4, 1998, pages 238 - 241, XP008095119
- WANG W. ET AL.: 'Effects of musk glucoprotein on PAF production' CHINA JOURNAL OF CHINESE MATERIA MEDICA. vol. 25, no. 12, December 2000, pages 733 - 736, XP008071106
- WANG W ET AL.: 'Effects of musk glucoprotein on the function' CHINA JOURNAL OF CHINESE MATERIA MEDICA. vol. 26, no. 1, January 2001, pages 50 - 52, XP008071131
- WANG W. ET AL: 'Effects of musk glucorotein on chemotaxis of polymorphon' CHINA JOURNAL OF CHINESE MATERIA MEDICA. vol. 28, no. 1, January 2003, pages 59 - 62, XP008095120
- MENG Z. ET AL.: 'Impact of subcutaneously embedded musk on the' CHINA TUMOR CLINIC vol. 25, no. 11, 1998, pages 834 - 836, XP008095118

## Description

### FIELD OF THE INVENTION

The present invention relates to an amino acid sequence from the active principles in traditional Chinese medicine musk and their acetic salts.

### BACKGROUND OF THE INVENTION

Identification of the active principles in traditional Chinese medicine (TCM) is a prerequisite for modernization of TCM. We have identified the main components and active principles in hundreds of commonly used medical herbs or natural products and the medical effects of many of the active principles. However, the research in this aspect, in general, is far from being complete. For example, many components identified have not been correlated with clinical effects. The fundamental theories and the research techniques in the active principles of TCM need to be improved, and new concepts and novel technology need to be introduced. Nowadays, with the development of gene technology, proteins and peptides in TCM are should be the focus for the research of active principles in TCM.

For a long time, the commonly used methods to identify the active principles, such as flavones, sterol or glycoside, in TCM have been limited to chemical or physical analysis. Researchers have not paid much attention to peptides and proteins. Proteins and peptides are important components various biological functions. Research on bioactive peptides and their related genes may lead to discovery of novel therapeutics or medicinal precursors.

Research has shown that musk contains about 25% protein, but the molecular structure of musk's polypeptide has not been reported yet. At present, some chemical compositions such as muscone, muscopyridine, C 19 steroid can be synthesized.

During the Sixth and Seventh Five-year Plan periods, our government organized a series of key scientific researches to obtain the composition spectrum of natural musk and reconstruct synthesized musk. Our researchers have obtained from natural musk the musk's polypeptide, which polypeptide comprises 15 amino acid, and have demonstrated that the inhibitive effect of musk's polypeptides to inflammation in rat's ear caused by croton oil is 36 times (based on mg/kg) or 500 times (based on molarity) stronger than that of hydrocortisone. It has been proven that musk polypeptides are indispensable ingredients for musk to exert pharmaceutical effects. However, when TCM musk was submitted for approval as a new medicine, it was replaced by some artificial product (e.g., fanghuosu) because of the failure to synthesize musk's polypeptides. This is one of the reasons that people still prefer natural musk.

Acta Pharm. Sinica, 1988, 23(6), 406-410 discloses the anti-inflammatory activity of a polypeptide isolated from the water soluble fraction of an extract of musk. WO-A-0226781 discloses various polypeptide sequences.

According to the fact that musk's polypeptides have stronger effect than glucocorticoids, it can be speculated that musk's polypeptides may have important therapeutic effects for immune-mediated tissue injuries and diseases, such as allergic asthma, glomerulonephritis, systemic lupus erythematosus, rheumatoid arthritis, pustular vasculitis, myasthenia gravis, diabetes, etc. To date, these injuries and diseases are still treated with glucocorticoids, which lead to toxic side effects caused by steroid hormone. Therefore, it is still very difficult to treat these diseases. In general, musk's polypeptides have great values in clinical application and a huge market prospect.

### OBJECTIVE OF THE INVENTION

The aim of the invention is to provide polypeptide sequences obtained from the active principles in TCM musk and their preparation method.

### TECHNIQUES USED IN THE INVENTION

In order to utilize the active principles from musk as medicines, the invention provides amino acid sequences obtained from the active principles in TCM musk such as:
SDSECPLLCEVWILK
SDSECPLLPRQGTGSLH
IDCECPLLEAKCPSFPLWPQGREERQ
SDSECPLLLNGTNTSSRFESINCVFLSTEEGC
and their respective acetic salts such as:
(SDSECPLLCEVWILK)Ac
(SDSECPLLPRQGTGSLH)Ac
(IDCECPLLEAKCPSFPLWPQGREERQ)Ac
(SDSECPLLLNGTNTSSRFESINCVFLSTEEGC)Ac

The invention also provides a method to obtain amino acid sequences from the active principles in TCM, characterized in: separating and purifying the protein and polypeptide extracted from the functionally active parts that contain active principles of TCM and obtaining active polypeptides or proteins of pharmaceutical values; determining the effect through pharmacodynamical analysis and identifying the amino acid sequences; constructing a cDNA library from the active parts or tissues of animals and plants and obtaining the target genes that encode the active peptides; and finally obtaining the amino acid sequences of active polypeptides.

The purification and separation as disclosed *supra* means using the proteins and polypeptides separation and purification techniques (such as Affinity Chromatogram, Hydrophobic Interaction Chromatogram, Gel Electrophoresis, HPLC, Mass Spectrometry, etc.) to extract the proteins and polypeptides, whose active parts reflect the active principles in TCM, then analyzing the pharmacodynamical effect of the proteins and polypeptides through pharmaceutical tests to identify their pharmaceutical values, and identifying the amino acid sequences.

To construct a cDNA library is to collect the tissues of animals and plants containing active principles, extract mRNA or total RNA from cells, obtain cDNA using the RT-PCR technique, clone total cDNA fragments to the expression carrier. Theoretically, a cDNA library is a collection of the total clones of different cDNA fragments or total gene fragments in a cell. From cDNA library or from mRNA directly, the gene encoding polypeptide and protein in TCM can be obtained by using PCR technique, and amino acid sequence can be deduced after the gene have been sequenced and verified. On one hand, polypeptide can be synthesized by chemical methods; on the other hand, by cloning the gene sequence to the prokaryotic or eukaryotic expression carrier, constructing a recombinant plasmid, transforming host cells, obtaining engineered bacteria through various screening markers, the recombinant active polypeptides and proteins can be obtained by fermentation and purification of the engineered bacteria.

The gene of the active principles in TCM is the gene that encodes musk's polypeptides. Musk is the dried secretion obtained from the musk gland of a musk deer (Muschus berezovskii Flerov). It is a commonly used precious traditional Chinese medicine and has 2000 years of history used to cure disease. Musk ranked at the top in Shen Long Ben Cao Jing, the oldest classic of Chinese medicine, and was recorded in the medical books over the past dynasties. Musk has the functions of reviving spirit, activating blood circulation, relieving swelling and pains. It shows very significant effect in quite small doses and can be used to cure some emergencies, such as coma, pain or inflammation. In recent years, although remarkable progress has been made in the preparation of artificial musk and its pharmaceutical effect in China, researchers have focused on synthesizing some basic chemical compositions like muscone rather than the active principles in musk, musk's polypeptides and their gene. Synthesizing musk's polypeptides by chemical techniques or gene engineering technology can overcome the limits of using fanghuosu as the substitute, preserve and exploit the gene resources of TCM.

### DESCRIPTION OF THE FIGURES

FIG. 1: Chromatogram analysis of musk peptides of the invention
FIG. 2: One strip separated from the sequence in Figure 1
FIG. 3: Mass spectography of SDSECPLLCEVWILK
FIG. 4: Mass spectography of SDSECPLLPRQGTGSLH
FIG. 5: Mass spectography of IDCECPLLEAKCPSFPLWPQGREERQ
FIG. 6: Mass spectography of SDSECPLLLNGTNTSSRFESINCVFLSTEEGC
FIG. 7: Technology flowchart of amino acid sequence acquisition

### EXAMPLES

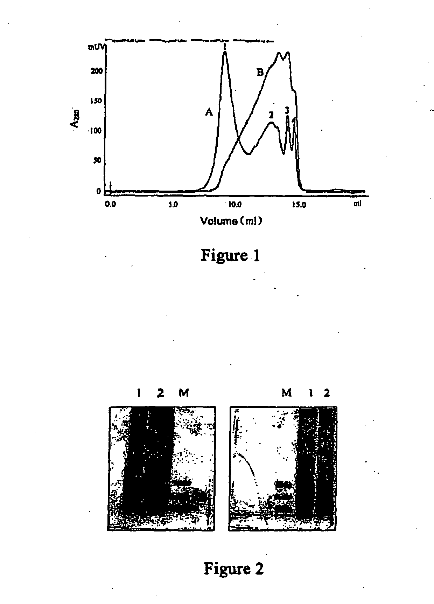

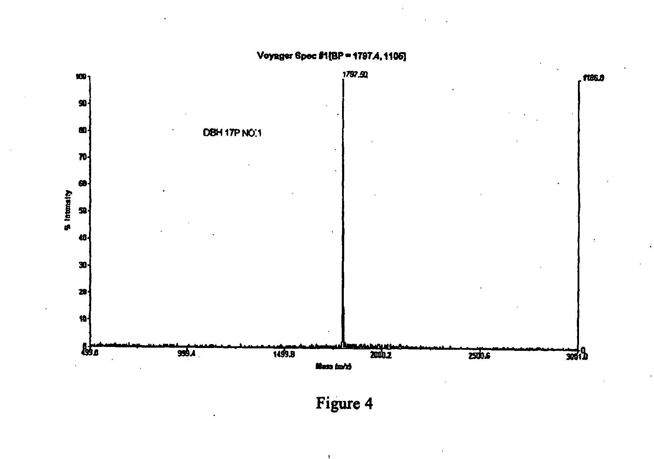

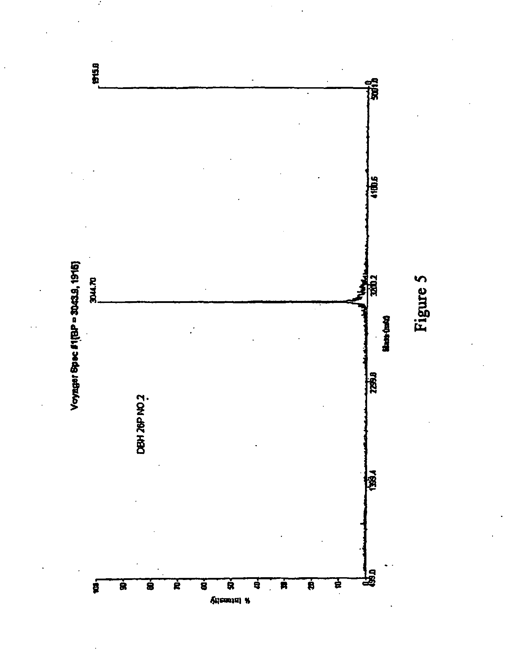

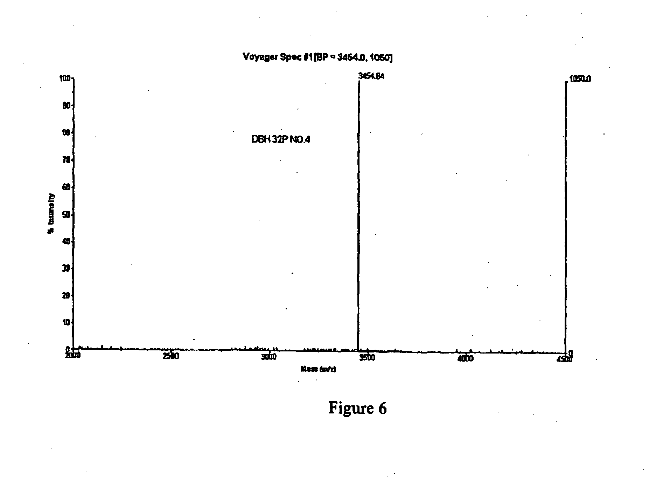

### See FIGs.1-6

### Synthesis and pharmaceutical study of musk's active polypeptides

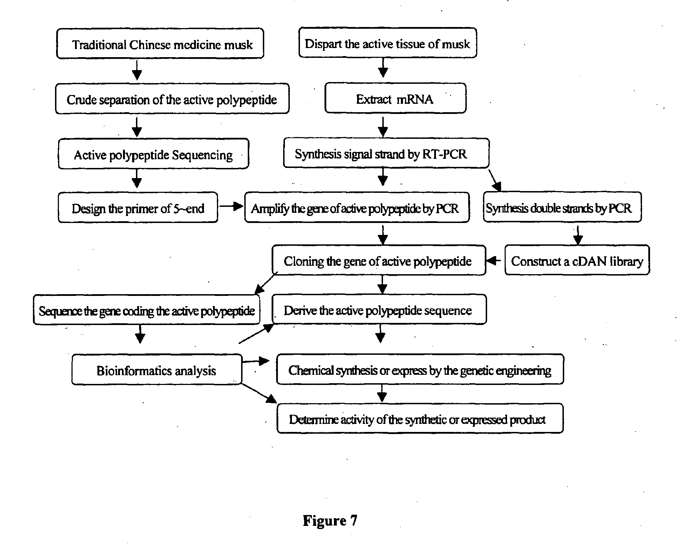

In order to first determine the target substance, the water-soluble part of natural musk was separated and purified and its pharmaceutical effects were analyzed in the study. Details of the procedures, as presented in FIG. 7, are explained as follows.

### 1. Chromatogram analysis (FIG. 1) and gel electrophoresis analysis (FIG. 2)

Natural musk, after extracted using organic solvents such as 95% ethanol, was dissolved in sterile water and prepared into samples for analysis. The supernatant of the sample was taken and filtered with a membrane of 0.22 µm after the sample was centrifuged. Tricine SDS-PAGE was conducted and the results were determined by means of silver staining. The results showed the basic molecular weight of proteins or peptides present in natural musk, which served as the basis for planning subsequent experiments. In this study, chromatogram was conducted on the parts under 8 kDa. The chromatogram column was Superdex Peptide Hr 10/30(Pharmacia); the column was equilibrated by two bed volumes of ddH2O; 200 µL of the sample was loaded to the column; 1.5 bed volumes of ddH2O was used to wash the column with a flow rate of 0.5ml/min. All 4 eluted peaks were collected (Figure 1. A: UV absorption Curve; B: Conductance Curve: 1: Elution Peak 1; 2: Elution Peak 2; 3: Elution Peak 3; 4: Elution Peak 4), which primarily showed that there were 4 groups in this molecular weight range. After pharmaceutical studies were conducted for the 4 groups respectively and electrophoresis was conducted for those groups that definitely had pharmaceutical effects, single bands obtained (Figure 2. 1 and 2: Sample Analysis; M: Protein molecular weight meter) were used for amino acid sequence analysis.

### 2. HPLC analysis

Predicted eluted peak components can be analyzed by high-pressure liquid chromatogram (HPLC) to further find out whether each of the groups is composed of a single component. If they are of multiple components, HPLC can be conducted to further separate and purify the components until single component is obtained. The single component thus obtained can be directly used in analyzing amino acid sequences. It can also be used for further mass spectrum if needed.

### 3. Mass spectrum and amino acid sequence measurement

Mass spectrum technology can be used to examine the single component obtained so as to obtain the data of components and sequences of amino acid. By means of further analysis of amino sequences, the terminal or complete sequence of amino acid can be determined. As a result, the encoded gene sequence can be deduced and used in chemical synthesis and genetic engineering production.

### 4. Obtaining the gene sequence

The method described *supra* to deduce gene sequences can lead to many possible sequences due to the degenerative codons. The problem can be solved through the following two ways:
a) Based on the selected expression system and the codons bias of the host cell, design and synthesize a target gene sequence, identify the sequence, and verify the synthesized sequence.
b) According to the deduced gene sequence, synthesize a primer, obtain the gene encoding the protein or polypeptide of musk deer. First, collect the musk glands of forest musk deer, then extract their mRNA and conduct RT-PCR. The cDNA obtained can be used to construct a cDNA library, or directly obtain the target gene sequence by means of PCR.

### 5. Obtaining the amino acid sequence

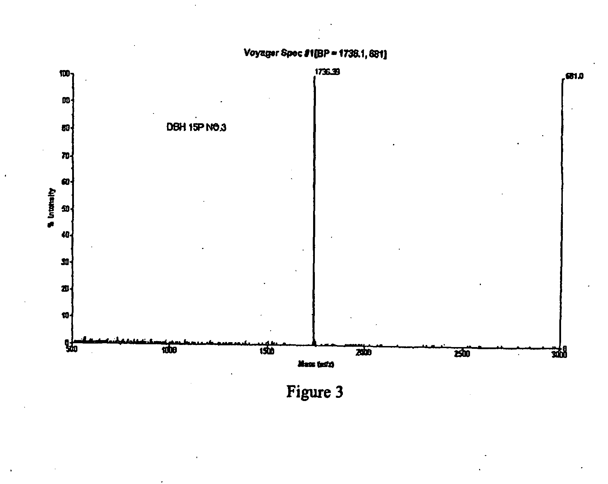

A number of amino acid sequences were obtained from the above mentioned gene sequence. By utilizing biological information technology, candidate sequences were selected according to the pharmaceutical effects. The candidate sequences were chemically synthesized by the CL. (XIAN) BIO.SCIENTIFIC CO., LTD. The quality of the synthesis was confirmed by mass spectrometry (Figures 3-6) The synthesized polypeptide sequences include:
SDSECPLLCEVWILK
SDSECPLLPRQGTGSLH
IDCECPLLEAKCPSFPLWPQGREERQ
SDSECPLLLNGTNTSSRFESINCVFLSTEEGC

Comparison of the four polypeptide sequences shows that each sequence comprises **ECPLL**, and polypeptide comprising more than 30% of conserved segments with the above sequences had the same properties of anti-inflammation and immune-suppressive activeness. For instance, in the first sequence SDSECPLLCEVWILK and the second one SDSECPLLPRQGTGSLH, as far as the identicalness of the two sequences is concerned, the former had 53% conserved, and latter had 47% conserved. Comparison of identicalness of the first sequence SDSECPLLCEVWILK and the third sequence IDCECPLLEAKCPSFPLWPQGREERQ showed that the former had 53% conserved and that the latter had 31% conserved. Comparison of the first sequence SDSECPLLCEVWILK and the fourth one SDSECPLLLNGTNTSSRFESINCVFLSTEEGC showed that the former had 73% conserved and the latter had 34% conserved.

The polypeptides related to the above sequences can form various forms of salts, of which acetates are the most common. Data shows that acetates play certain role in improving solubility and activity and usually contain 10-15% of acetic acid. Such polypeptide acetic salts include:
(SDSECPLLCEVWILK) Ac
(SDSECPLLPRQGTGSLH) Ac
(IDCECPLLEAKCPSFPLWPQGREERQ) Ac
(SDSECPLLLNGTNTSSRFESINCVFLSTEEGC) Ac

### 6. A laboratory study of the pharmaceutical effects of polypeptide syntheses

### (1) A laboratory study on the auricular inflammation in mice - inducing auricular swelling in mice with dimethylbenzene:

The subjects were Class II K.M mice, all male, weighing 20-24g, provided by the Animal Experiment Center of the Zhongshan University. There was a model control group, positive (hydroxycorticoids) control group and sample group. The suppressing function of the sample on the dimethylbenzene-induced acute inflammation was observed. The degree of ear swelling was the index. 100µg of the sample, diluted with sterile PBS to a concentration needed, was given through IV injection. The result showed that the sample had significant suppressive effect on ear swelling in mice, which proves that the above active peptide is significantly active in anti-inflammation.

### (2) Laboratory study on LPS-induced inflammation in mice

A model control group, positive (hydroxycorticoids) control group and sample group were formed. It was observed that the sample had suppressing effect on the activation, biosynthesis and release of inflammation-related cytokines secreted by animals that had LPS-induced early acute inflammation. Balb/c mice were provided by the Animal Experiment Center of the Zhongshan University. ELISA method was used to measure the cytokines in the mice. The dose of the sample was 25~100 µg. The results showed that active polypeptide had significant function of suppressing cytokines (IL-6, TNF-α, IFN-γ) related to inflammation. And the suppressive effect of the sample was 500 times stronger than that of glucocorticoids in suppressing inflammatory cytokines, which proves that the medicine under discussion has the function of suppressing inflammation and autoimmune disorders.

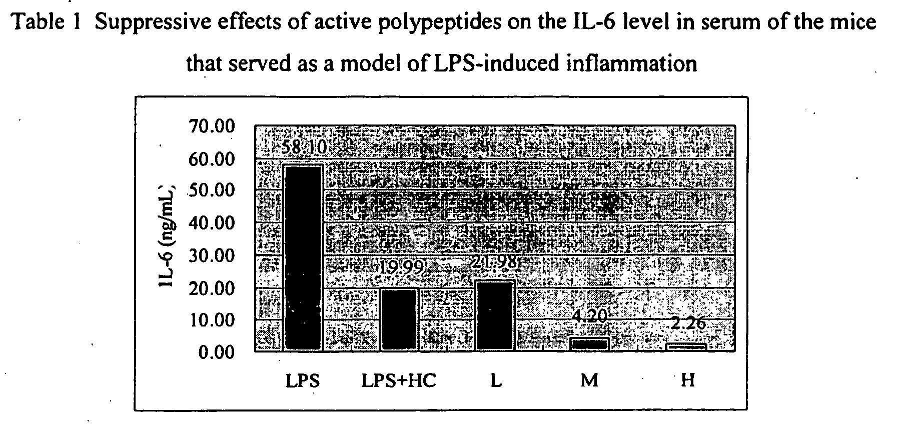

A dose of 2.5mg/Kg of LPS (Sigma) was injected through intraperitoneal injection. At 0.5 hour after the injection, 5 groups were formed randomly: (1) negative control group (LPS), which received saline injection through intraperitoneal injection; (2) positive control group (LPS+H.C), which received 2.5mg/kg of hydroxycorticoid through intraperitoneal injection; (3) low-dose active peptide group (L), which received a 2.5µg/kg intraperitoneal injection; (4) medium-dose active peptide group, which received a 50µg/kg intraperitoneal injection; and (5) high-dose active peptide group, which received a 100µg/kg intraperitoneal injection. At 2 hours, ELISA was used to measure the IL-6 level in the serum of all groups.

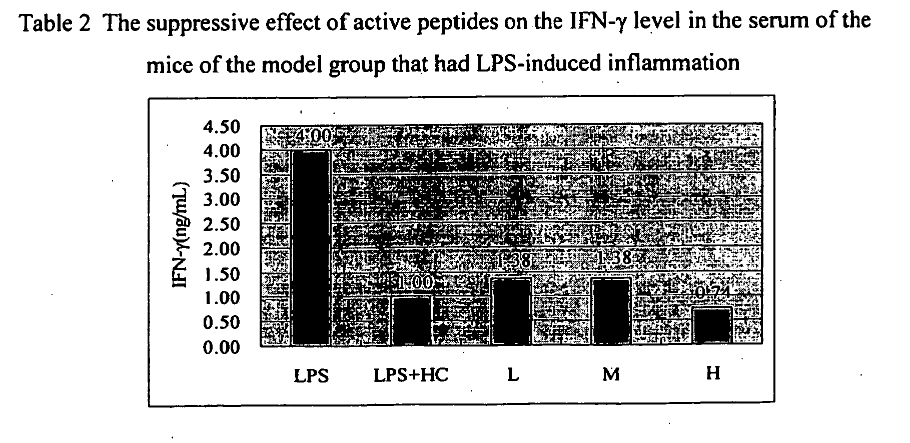

A dose of 2.5mg/Kg of bacteriotoxin (Sigma) was injected through intraperitoneal injection. At 0.5 hour after the injection, 5 groups were formed randomly: (1) negative control group (LPS), which received saline injection through intraperitoneal injection; (2) positive control group (LPS+H.C), which received 2.5mg/Kg of hydroxycorticoid through intraperitoneal injection; (3) low-dose active peptide group (L), which received a 2.5µg/Kg intraperitoneal injection; (4) medium-dose active peptide group, which received a 50µg/kg intraperitoneal injection; and (5) high-dose active peptide group, which received a 100µg/Kg intraperitoneal injection. At 2 hours, ELISA was used to measure the IFN-γ level in the serum of all groups.

### (3) A laboratory study on adjuvant arthritis in rats

This is a model of T-cell-mediated autoimmune disorders. This model has often been used in the study of pain relievers and immuno-suppressive drugs. Freund's FCA was injected percutaneously in the right side heel planta. First, local acute inflammation appeared and followed by delayed systemic metamorphic changes, resulting in polyarthritis. Test indexes: foot swelling, auricular erythema and inflammatory nodules in the tail, and weight change. Class II male rats were used, weighing 180±20g, provided by the First Military Medical University Animal Experimental Center (Certificate No. 0000830, 0000858), Indommethacin as positive control. Results showed that the sample has significant suppressive effect over arthritis with a functioning dose of 1.2~6 µg/kg.

### (4)Acute toxicity test in mice

Clean Class NIH-type mice (certificate no. 2002A022), were used, half male and half female, weighing 20~22g, provided by the Guandong Medical Animal Experiment Center. One-off IV injection was done in the tail of the mice and followed by immediate observation of the mice for acute toxicity reaction. The test was repeated for a week. No mouse died and all mice showed no signs of toxicity. Results showed LD₅₀ >5mg/kg, indicating that the sample is safe.

### (5) Neurodermatitis

Volunteers who had chronically had neurodermatitis used the sample and responded well.

## Claims

1. A peptide isolated from the active principles of natural musk, the peptide comprising SDSECPLLCEVWILK, and its acetate salt (SDSECPLLCEVWILK) Ac.

2. A peptide isolated from the active principles of natural musk, the peptide comprising SDSECPLLPRQGTGSLH, and its acetate salt (SDSECPLLPRQGTGSLH) Ac.

3. A peptide isolated from the active principles of natural musk, the peptide comprising IDCECPLLEAKCPSFPLWPQGREERQ, and its acetate salt (IDCECPLLEAKCPSFPLWPQGREERQ) Ac.

4. A peptide isolated from the active principles of natural musk, the peptide comprising SDSECPLLLNGTNTSSRFESINCVFLSTEEGC, and its acetate salt (SDSECPLLLNGTNTSSRFESINCVFLSTEEGC) Ac.

5. Peptide as claimed in claim 1, 2, 3 or 4 for use as an anti-inflammatory drug or immunological inhibitor.

6. A method of preparing the peptide of claim 1, 2, 3, or 4, the method comprising: separating and purifying the peptide to obtain the active peptide or protein of pharmaceutical value; determining their pharmaceutical effects by means of pharmacodynamical analysis; identifying the amino acid sequences; then, constructing a cDNA library using active components or tissues from animals or plants to obtain target genes encoding the peptide; obtaining the amino acid sequences of the peptide.

## Patentansprüche

1. Peptid, das aus den Wirkstoffen von natürlichem Moschus isoliert wurde, wobei das Peptid SDSECPLLCEVWILK umfasst, und sein Acetatsalz (SDSECPLLCEVWILK) Ac.

2. Peptid, das aus den Wirkstoffen von natürlichem Moschus isoliert wurde, wobei das Peptid SDSECPLLPRQGTGSLH umfasst, und sein Acetatsalz (SDSECPLLPRQGTGSLH) Ac.

3. Peptid, das aus den Wirkstoffen von natürlichem Moschus isoliert wurde, wobei das Peptid IDCECPLLEAKCPSFPLWPQGREERQ umfasst, und sein Acetatsalz (IDCECPLLEAKCPSFPLWPQGREERQ) Ac.

4. Peptid, das aus den Wirkstoffen von natürlichem Moschus isoliert wurde, wobei das Peptid SDSECPLLLNGTNTSSRFESINCVFLSTEEGC umfasst, und sein Acetatsalz (SDSECPLLLNGTNTSSRFESINCVFLSTEEGC) Ac.

5. Peptid, wie es in Anspruch 1, 2, 3 oder 4 beansprucht ist, zur Verwendung als antiinflammatorischer Wirkstoff oder immunologischer Inhibitor.

6. Verfahren zur Herstellung des Peptids gemäß Anspruch 1, 2, 3 oder 4, wobei das Verfahren umfasst: Abtrennen und Reinigen des Peptids unter Erhalt des aktiven Peptids oder Proteins von pharmazeutischem Wert; Bestimmen seiner pharmazeutischen Wirkungen mittels pharmakodynamischer Analyse; Identifizierung der Aminosäuresequenzen; danach Konstruieren einer cDNA-Bibliothek unter Verwendung von aktiven Komponenten oder Geweben aus Tieren oder Pflanzen, um Target-Gene zu erhalten, die das Peptid codieren; Erhalten der Aminosäuresequenzen des Peptids.

## Revendications

1. Peptide isolé à partir des principes actifs du musc naturel, le peptide comprenant SDSECPLLCEVWILK, et son sel d'acétate (SDSECPLLCEVWILK) Ac.

2. Peptide isolé à partir des principes actifs du musc naturel, le peptide comprenant SDSECPLLPRQGTGSLH, et son sel d'acétate (SDSECPLLPRQGTGSLH) Ac.

3. Peptide isolé à partir des principes actifs du musc naturel, le peptide comprenant IDCECPLLEAKCPSFPLWPQGREERQ, et son sel d'acétate (IDCECPLLEAKCPSFPLWPQGREERQ) Ac.

4. Peptide isolé à partir des principes actifs du musc naturel, le peptide comprenant SDSECPLLLNGTNTSSRFESINCVFLSTEEGC, et son sel d'acétate (SDSECPLLLNGTNTSSRFESINCVFLSTEEGC) Ac.

5. Peptide selon la revendication 1, 2, 3 ou 4, utilisable comme médicament anti-inflammatoire ou inhibiteur immunologique.

6. Procédé de préparation du peptide selon la revendication 1, 2, 3 ou 4, le procédé comprenant : la séparation et la purification du peptide pour obtenir le peptide actif ou une protéine présentant un intérêt pharmaceutique ; la détermination de leurs effets pharmaceutiques au moyen d'une analyse pharmacodynamique ; l'identification des séquences d'acides aminés ; puis la construction d'une banque d'ADNc au moyen de composants actifs ou de tissus provenant d'animaux ou de plantes pour obtenir des gènes cibles codant le peptide ; l'obtention des séquences d'acides aminés du peptide.
